(19) Europäisches Patentamt
European Patent Office
Office européen des brevets

(11) **EP 4 616 797 A1**

(12) **EUROPEAN PATENT APPLICATION**
published in accordance with Art. 153(4) EPC

(43) Date of publication:
**17.09.2025  Bulletin 2025/38**

(21) Application number: **23887913.4**

(22) Date of filing: **03.11.2023**

(51) International Patent Classification (IPC):
**A61B 5/145** (2006.01)    **A61N 5/10** (2006.01)
**G01T 1/02** (2006.01)    **G16H 20/40** (2018.01)

(52) Cooperative Patent Classification (CPC):
**A61B 5/145; A61N 5/10; G01T 1/02; G16H 20/40**

(86) International application number:
**PCT/CN2023/129479**

(87) International publication number:
**WO 2024/099223 (16.05.2024 Gazette 2024/20)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **07.11.2022  CN 202211386003
26.10.2023  CN 202311398156**

(71) Applicant: **Neuboron Therapy System Ltd.
Xiamen, Fujian 361026 (CN)**

(72) Inventors:
• **QIU, Honglin
Nanjing, Jiangsu 211112 (CN)**
• **TENG, Yi-chiao
Nanjing, Jiangsu 211112 (CN)**
• **SHU, Diyun
Nanjing, Jiangsu 211112 (CN)**
• **LIU, Yuanhao
Nanjing, Jiangsu 211112 (CN)**

(74) Representative: **Gunzelmann, Rainer
Wuesthoff & Wuesthoff
Patentanwälte und Rechtsanwalt PartG mbB
Schweigerstraße 2
81541 München (DE)**

(54) **BORON NEUTRON CAPTURE TREATMENT SYSTEM AND IRRADIATION DOSE CORRECTION METHOD**

(57)    The present application relates to a boron neutron capture treatment system and an irradiation dose correction method. The boron neutron capture treatment system comprises a neutron beam irradiation module for generating a neutron beam, a blood boron concentration detection device for detecting the actual concentration of blood boron in an irradiated body, a treatment planning module for generating a preset treatment plan, an irradiation dose correction module for acquiring a corrected irradiation dose according to the actual concentration of blood boron, and a control module. The control module is configured for retrieving the preset treatment plan from the treatment planning module and controlling the irradiation time of the neutron beam irradiation module according to the corrected irradiation dose, such that an irradiation dose received by a patient reaches a target dose, thereby reducing the error of the irradiation dose caused by a change in the concentration of blood boron during actual irradiation.

FIG. 4

**Description**

**TECHNICAL FIELD**

An aspect of the present disclosure relates to a radiotherapy system, and in particular to a boron neutron capture therapy (BNCT) system. Another aspect of the present disclosure relates to an irradiation dose correction method, and in particular to an irradiation dose correction method for the BNCT system.

**BACKGROUND**

With the development of atomics, the radiotherapy such as the cobalt-60, the linear accelerator, and the electron beam has become one of major means to treat cancers. However, the conventional photon or electron therapy is restricted by physical conditions of radioactive rays. Specifically, while tumor cells are killed, a large number of normal tissues on a beam path are damaged. Due to different sensitivities of the tumor cells for the radioactive rays, the conventional radiotherapy is often undesirable to treat radioresistant malignant tumors (such as glioblastoma multiforme and melanoma).

In order to reduce radiation damages to the normal tissues surrounding the tumor, the target therapy in chemotherapy has been employed in the radiotherapy. For the highly radioresistant tumor cells, the radiotherapy with high relative biological effectiveness (RBE), including the proton therapy, the heavy particle therapy, and the neutron capture therapy, has also been developed actively. With specific aggregation of boron-containing drugs in tumor cells, and in cooperation with accurate neutron beam control, BNCT in neutron capture therapy serves as a better alternative to treat the cancers.

According to the BNCT, with a large capture cross section of the boron ($^{10}$B)-containing drug for thermal neutrons, and through the $^{10}$B(n,$\alpha$)$^7$Li neutron capture reaction and the nuclear fission reaction, $^4$He and $^7$Li heavy charged particles are generated. The two heavy charged particles have an average energy of about 2.33 MeV, and have characteristics of the high linear energy transfer (LET), and the short range. The alpha particle has the LET of 150 keV/$\mu$m, and the range of 8 $\mu$m. The $^7$Li heavy charged particle has the LET of 175 keV/$\mu$m, and the range of 5 $\mu$m. The total range of the two particles is approximately equivalent to a cell size, such that the radiation damage to an organism is limited to a cell level. When the boron-containing drug is aggregated to the tumor cells selectively, and an appropriate neutron source is provided, a purpose of locally killing the tumor cells on premise of no serious damage to normal tissues can be achieved.

In the BNCT, since the neutron beam for irradiating radioactive rays onto the irradiated body for treatment has the strong radioactive rays, an irradiation dose on the irradiated body is to be controlled accurately, so as to achieve the better treatment effect, and reduce the radiation damage of the radioactive rays on the irradiated body as much as possible. Hence, the accurate formulation of the treatment plan is crucial. In clinical practices, the boron-containing drugs such as the boronophenylalanine (BPA) in the BNCT are expensive in general. In order to save treatment expenses of the irradiated body and simplify the treatment process, when the treatment plan is formulated, a preset blood boron concentration is used for simulation and calculation to estimate the irradiation dose. In actual treatment, the boron-containing drug is injected into the irradiated body continuously. Before the irradiated body enters an irradiation room, blood of the irradiated body is drawn to measure an actual blood boron concentration of the irradiated body, thereby injecting the boron-containing drug continuously in irradiation to maintain the blood boron concentration.

The accurate irradiation dose of the neutron beam plays a crucial role in the actual treatment. The excessive irradiation dose will pose a potential injury to the irradiated body, while the insufficient irradiation dose will affect the treatment quality. The irradiation dose of the neutron beam depends on the blood boron concentration and a neutron dose of the neutron beam actually irradiated onto the irradiated body. In the actual treatment, it is a common practice to perform simulation and calculation according to the preset boron concentration to obtain a preset treatment plan. Different irradiated bodies have different metabolic statuses. In addition, the blood boron concentration of the irradiated body is varied for different drugs and different injection manners, resulting in a difference between the preset blood boron concentration and the actual blood boron concentration of the irradiated body. Therefore, there is an error in the preset treatment plan formulated according to the preset blood boron concentration. It is desired to provide a BNCT system capable of correcting the preset treatment plan to ensure the treatment effect, and an irradiation dose correction method.

**SUMMARY**

In view of the above-mentioned technical problem, the present disclosure provides a BNCT system capable of ensuring a treatment effect and an irradiation dose correction method.

According to an aspect, the present disclosure provides a BNCT system, including: a neutron beam irradiation module configured to generate a neutron beam; a blood boron concentration detecting device configured to detect an actual blood boron concentration of an irradiated body; a treatment planning module configured to generate a preset treatment plan; an irradiation dose correction module configured to acquire a corrected irradiation dose according to the actual blood boron concentration; and a control module configured to invoke the preset treatment plan from the treatment planning module, and control irradiation time of the neutron beam irradiation module according to the corrected irradiation dose, such that an irradiation dose received by a patient reaches a target dose.

Further, the treatment planning module is configured to perform simulation and calculation according to a preset blood boron concentration to obtain the preset treatment plan.

Further, the irradiation dose correction module is configured to correct a target dose rate according to the actual blood boron concentration to obtain the corrected irradiation dose.

Further, the BNCT system further includes a neutron dose detecting device configured to detect a cumulative neutron count in real time to obtain the irradiation dose.

Further, the neutron dose detecting device is a $BF_3$ proportional counter.

Further, a preset cumulative neutron count $N_{preset}$ is calculated by:

$$N_{preset} = R_{BF3,cal} \times T_{preset} \tag{1}$$

where, $R_{BF3,cal}$ is a theoretical count rate of the neutron dose detecting device, $B_{preset}$ is the preset boron concentration, $T_{preset}$ is preset irradiation time corresponding to the preset boron concentration $B_{preset}$, and $T_{preset}$ is calculated by:

$$T_{preset} = \frac{D_{ROI,prescribed}}{\dot{D}_{ROI}(B_{preset} \times TBR)} \tag{2}$$

where, $D_{ROI,prescribed}$ is the target dose, $\dot{D}_{ROI}(B_{preset} \times TBR)$ is a preset target dose rate, and $\dot{D}_{ROI}(B_{preset} \times TBR)$ is calculated by:

$$\dot{D}_{ROI}(B_{preset} \times TBR) = aB_{preset}^2 + bB_{preset} + c \tag{3}$$

where, a, b, and c each are a fitting coefficient, which is obtained through function fitting on a plurality of preset boron concentrations and corresponding simulated dose rates.

Further, a corrected cumulative neutron count $N_{update}$ is calculated by:

$$N_{update} = R_{BF3,cal} \times T_{update} = R_{BF3,cal} \times \frac{D_{ROI,prescribed}}{\dot{D}_{ROI}(B_{update} \times TBR)} \tag{4}$$

where, $B_{update}$ is the actual blood boron concentration, $T_{update}$ is a planned irradiation time corresponding to the actual blood boron concentration $B_{update}$, $\dot{D}_{ROI}(B_{update} \times TBR)$ is a corrected target dose rate corresponding to the actual blood boron concentration $B_{update}$, and $\dot{D}_{ROI}(B_{update} \times TBR)$ is calculated by:

$$\dot{D}_{ROI}(B_{update} \times TBR) = aB_{update}^2 + bB_{update} + c \tag{5}$$

where, a, b, and c each are a fitting coefficient, which is obtained through function fitting on a plurality of preset boron concentrations and corresponding simulated dose rates.

According to another aspect, the present disclosure further provides an irradiation dose correction method for a BNCT system, including: generating a preset treatment plan in combination with a preset boron concentration and medical image data of an irradiated body; acquiring a corrected irradiation dose according to an actual boron concentration; and

controlling irradiation time according to the corrected irradiation dose, such that an irradiation dose received by a patient reaches a target dose.

Further, the irradiation dose correction method further includes: determining the target dose based on the medical image data of the irradiated body.

Further, the irradiation dose correction method further includes: acquiring a preset irradiation dose.

Further, acquiring the corrected irradiation dose specifically includes: correcting a preset target dose rate in the preset treatment plan according to the actually detected boron concentration to obtain a corrected target dose rate; and performing calculation according to the corrected target dose rate to obtain the corrected irradiation dose.

Further, a preset cumulative neutron count $N_{preset}$ is calculated by:

$$N_{preset} = R_{BF3,cal} \times T_{preset} \qquad (1)$$

where, $R_{BF3,cal}$ is a theoretical count rate of a neutron dose detecting device, $B_{preset}$ is the preset boron concentration, $T_{preset}$ is preset irradiation time corresponding to the preset boron concentration $B_{preset}$, and $T_{preset}$ is calculated by:

$$T_{preset} = \frac{D_{ROI,prescribed}}{\dot{D}_{ROI}(B_{preset} \times TBR)} \qquad (2)$$

where, $D_{ROI,prescribed}$ is the target dose, $\dot{D}_{ROI}(B_{preset} \times TBR)$ is a preset target dose rate, and $\dot{D}_{ROI}(B_{preset} \times TBR)$ is calculated by:

$$\dot{D}_{ROI}(B_{preset} \times TBR) = aB_{preset}^2 + bB_{preset} + c \qquad (3)$$

where, a, b, and c each are a fitting coefficient, which is obtained through function fitting on a plurality of preset boron concentrations and corresponding simulated dose rates.

Further, a corrected cumulative neutron count $N_{update}$ is calculated by:

$$N_{update} = R_{BF3,cal} \times T_{update} = R_{BF3,cal} \times \frac{D_{ROI,prescribed}}{\dot{D}_{ROI}(B_{update} \times TBR)} \qquad (4)$$

where, $B_{update}$ is the actual blood boron concentration, $T_{update}$ is a planned irradiation time corresponding to the actual blood boron concentration $B_{update}$, $D_{ROI}(B_{update} \times TBR)$ is the corrected target dose rate corresponding to the actual blood boron concentration $B_{update}$, and $\dot{D}_{ROI}(B_{update} \times TBR)$ is calculated by:

$$\dot{D}_{ROI}(B_{update} \times TBR) = aB_{update}^2 + bB_{update} + c \qquad (5)$$

where, a, b, and c each are a fitting coefficient, which is obtained through function fitting on a plurality of preset boron concentrations and corresponding simulated dose rates.

Further, the irradiation dose correction method further includes: providing a neutron dose detecting device configured to detect the irradiation dose in real time; and when the irradiation dose detected by the neutron dose detecting device reaches the corrected irradiation dose, controlling, by a control module, a neutron beam irradiation module to stop irradiation.

Further, the neutron dose detecting device is configured to acquire the irradiation dose by detecting a cumulative neutron count in real time, and is specifically a $BF_3$ proportional counter.

According to the irradiation dose correction method for a BNCT system provided by the embodiment of the present disclosure, by generating the preset treatment plan according to the preset blood boron concentration, correcting the preset target dose rate in the preset treatment plan according to the actual blood boron concentration to obtain the corrected target dose rate, performing the calculation according to the corrected target dose rate to obtain the corrected neutron dose, and controlling the irradiation time of the neutron irradiation module, there is no need to inject a boron-containing drug into the irradiated body when the preset treatment plan is formulated, as well as no need to implement the irradiation after the blood boron concentration is detected. This saves the treatment cost, and simplifies the treatment process. By correcting the irradiation dose according to the actual blood boron concentration in treatment, the irradiation dose correction method reduces an error between the actual irradiation dose and the target dose due to a change of the blood boron concentration in the actual irradiation.

## BRIEF DESCRIPTION OF THE DRAWINGS

FIG. 1 is a block diagram of a BNCT system according to an embodiment of the present disclosure;
FIG. 2 is a structural view of a layout of a BNCT system according to an embodiment of the present disclosure;
FIG. 3 is a schematic view of a beam shaping assembly (BSA) according to an embodiment of the present disclosure; and
FIG. 4 is a flowchart of an irradiation dose correction method according to an embodiment of the present disclosure.

In the figures: 100: BNCT system, 1: neutron beam irradiation module, 11: neutron generation device, 111: accelerator, 112: target, 12: BSA, 121: reflector, 122: moderator, 123: thermal neutron absorber, 124: radiation shield, 125: beam outlet, 13: collimator, 2: image acquisition module, 3: neutron dose detecting device, 4: blood boron concentration detecting device, 5: treatment planning module, 6: control module, 7: placement module, 8: irradiation dose correction module, and S: irradiated body.

## DETAILED DESCRIPTION OF THE EMBODIMENTS

To make the objectives, technical solutions, and advantages of the present disclosure clearer, the present disclosure is further described in detail below with reference to the drawings and embodiments. It should be understood that the specific embodiments described herein are merely used to explain the present disclosure, rather than to limit the present disclosure.

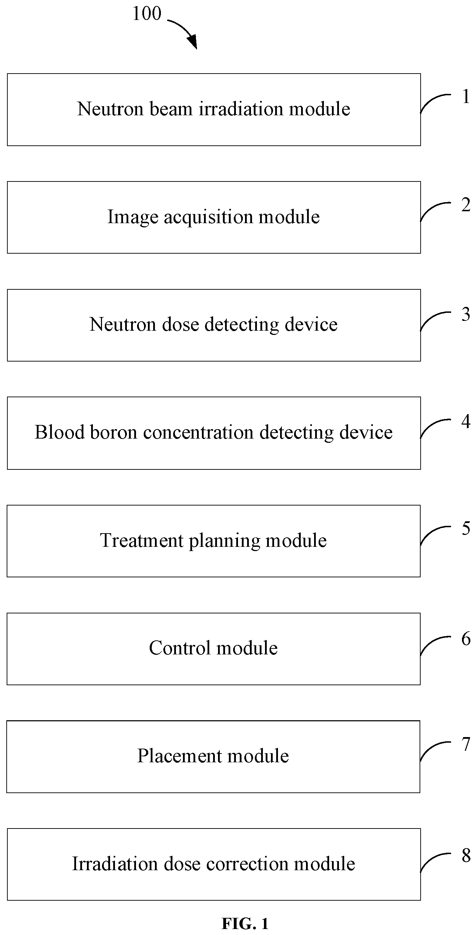

Referring to FIG. 1, a BNCT system 100 in the embodiment includes a neutron beam irradiation module 1, an image acquisition module 2, a neutron dose detecting device 3, a blood boron concentration detecting device 4, a treatment planning module 5, an irradiation dose correction module 8, a control module 6, and a placement module 7. Specifically, the neutron beam irradiation module 1 is configured to generate a neutron beam for treatment, and includes a neutron generation device 11, a BSA 12, and a collimator 13. The neutron generation device 11 is configured to generate the neutron beam. The BSA 12 is configured to adjust quality of the neutron beam generated by the neutron generation device 11, thereby reducing unnecessary dose deposition. The collimator 13 is configured to gather the neutron beam, such that the neutron beam has a high targeting ability in the treatment. The neutron dose detecting device 3 is configured to detect a neutron dose of the neutron beam generated by the neutron beam irradiation module 1. The blood boron concentration detecting device 4 is configured to detect an actual blood boron concentration of an irradiated body S. The treatment planning module 5 is configured to generate a preset treatment plan. The irradiation dose correction module is configured to acquire a corrected irradiation dose according to the actual blood boron concentration detected by the blood boron concentration detecting device 4. The control module 6 is configured to invoke the preset treatment plan of the irradiated body S at present from the treatment planning module 5, and control the neutron beam irradiation module 1 according to the corrected irradiation dose to implement irradiation. The placement module 7 is configured to place the irradiated body S.

The main principle of the BNCT is as follows: After the boron ($^{B-}10$)-containing drug is taken by or injected into the irradiated body S, the boron-containing drug is selectively gathered to the tumor cells M. With a large capture cross section of the boron ($^{B-}10$)-containing drug for thermal neutrons, and through the $^{10}B(n,\alpha)^7Li$ neutron capture reaction and the nuclear fission reaction, $^4He$ and $^7Li$ heavy charged particles are generated. The two heavy charged particles have an average energy of about 2.33 MeV, and have characteristics of the high LET, and the short range. The total range of the two particles is approximately equivalent to a cell size, such that the radiation damage to an organism can be limited to a cell level, and a purpose of locally killing the tumor cells on premise of no serious damage to normal tissues can be achieved.

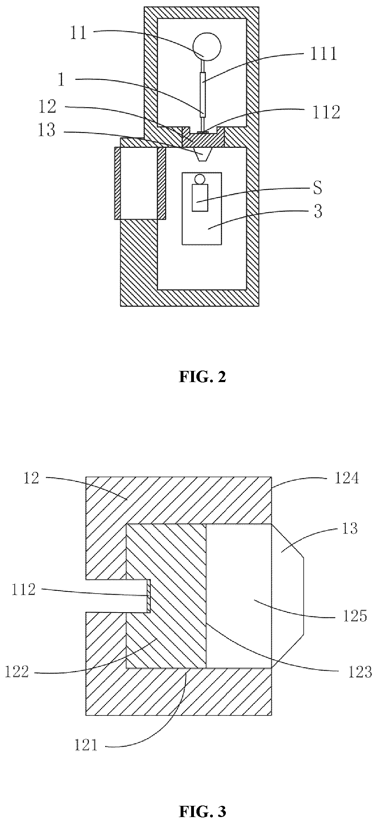

Referring to FIG. 2, in the embodiment of the present disclosure, the neutron generation device 11 includes an accelerator 111 and a target 112. The accelerator 111 is configured to accelerate charged particles (such as protons and deuterons) to generate a charged particle line such as a proton line. The charged particle line is irradiated onto the target 112 and interacted with the target 112 to generate a neutron line (a neutron beam). The target 112 is preferably a metal target 112. An appropriate nuclear reaction is selected according to characteristics such as a desired neutron yield and energy, available energies of the accelerated charged particles, a current, physical and chemical properties of the metal target 112, or the like. Nuclear reactions commonly discussed include $^7Li(p,n)^7Be$ and $^9Be(p,n)^9B$, both of which are endothermic reactions. In the embodiment of the present disclosure, the target 112 is made of lithium. However, as is known to those skilled in the art, the target 112 may also be made of a metal material other than the lithium and beryllium, such as tantalum (Ta) or tungsten (W). The target 112 may be a circular plate, may also be other solid shapes, and may further be a liquid (liquid metal). The accelerator may be a linear accelerator, a cyclotron, a synchrotron and a synchrocyclotron. In other implementations, the neutron generation device may be a nuclear reactor without the accelerator and the target.

No matter whether a neutron source in the BNCT comes from the nuclear reactor or the nuclear reaction between the accelerated charged particles and the target 112, a mixed radiation field is generated as a matter of fact, that is, the generated beam includes neutrons and photons having energies from low to high. As for the BNCT on deep-seated tumors, except the epithermal neutrons, the more the remaining radioactive rays, the greater the proportion causing non-selective dose deposition in the normal tissues. Therefore, the radioactive rays causing the unnecessary dose deposition should be reduced as much as possible. The BSA 12 can be configured to adjust the quality of the neutron beam generated by the neutron generation device 11, thereby reducing the unnecessary dose deposition. The collimator 13 is configured to gather the neutron beam, such that the neutron beam has the high targeting ability in the treatment.

Referring to FIG. 3, the BSA 12 includes a reflector 121, a moderator 122, a thermal neutron absorber 123, a radiation shield 124, and a beam outlet 125. The moderator 122 can be configured to adjust energies (>40 keV) of fast neutrons from the neutron generation device 11 to an energy range (0.5 eV to 40 keV) of epithermal neutrons, and reduce a content of thermal neutrons (<0.5 eV) as much as possible. The moderator 122 is made of a material having a large action cross section with the fast neutrons but a small action cross section with the epithermal neutrons. As a preferred embodiment, the moderator 122 is made of at least one of $D_2O$, $AlF_3$, Fluental™, $CaF_2$, $Li_2CO_3$, $MgF_2$, and $Al_2O_3$. The reflector 121 surrounds the moderator 122, and is configured to reflect neutrons diffused through the moderator 122 back to the neutron beam, thereby improving the utilization rate of the neutrons. The reflector is made of a material with a strong neutron reflectivity. As a preferred embodiment, the reflector 121 is made of at least one of Pb or Ni. On a transmission path of the neutron beam, the thermal neutron absorber 123 is provided behind the moderator 122, configured to absorb thermal neutrons passing through the moderator 122 to reduce the thermal neutrons in the neutron beam, and made of a material having a large action cross section with the thermal neutrons. As a preferred embodiment, the thermal neutron absorber 123 is made of $Li^{-6}$. In other embodiments, because of the $Li^{-6}$ in the material of the moderator 122, the thermal neutron absorber 123 may not be provided individually, but the moderator 122 serves as the thermal neutron absorber 123. The radiation shield 124 is configured to shield neutrons and photons leaked from a portion other than the beam outlet 125. A material of the radiation shield 124 includes at least one of a photon shielding material and a neutron shielding material. As a preferred embodiment, the material of the radiation shield 124 includes lead (Pb) as the photon shielding material and polyethylene (PE) as the neutron shielding material.

The collimator 13 is provided behind the beam outlet 125. An epithermal neutron beam from the collimator 13 is irradiated onto the irradiated body S. After passing through a superficial normal tissue of the irradiated body S, the epithermal neutron beam is moderated as thermal neutrons to reach the tumor cells for treatment.

It may be understood that the BSA 12 may also be other structures, provided that the epithermal neutron beam can be obtained to meet the treatment requirement. In the present disclosure, the collimator 13 may also not be provided, and the beam from the beam outlet 125 of the BSA 12 is directly irradiated onto the irradiated body S. For ease of description, when the collimator 13 is provided, an outlet of the collimator 13 may also be understood as the beam outlet 125.

The device for acquiring the three-dimensional (3D) medical image may be an imaging device such as a computed tomography (CT) device, a magnetic resonance imaging (MRI) device, a positron emission tomography (PET) device and an ultrasound device. In the present disclosure, preferably, the image acquisition module 2 of the CT device is used to acquire the medical image data of the irradiated body S through CT. The medical image data of the irradiated body S includes a coordinate matrix and a CT value matrix of a medical image voxel model of a to-be-irradiated portion (a lesion, namely the tumor cells) in a medical image coordinate system.

The neutron dose detecting device 3 includes a detector configured to receive neutrons and output a signal, a signal processing unit configured to process the signal output from the detector, a counter configured to count a signal output from the signal processing unit to obtain a count rate, a conversion unit configured to convert the count rate recorded by the counter into a neutron flux rate or a neutron dose rate, a calculation unit configured to perform integral calculation on the neutron flux rate or the neutron dose rate to obtain a neutron dose, and a display configured to display the neutron dose. In the solution, the neutron dose detected by the neutron dose detecting device 3 is the irradiation dose received by the irradiated body S.

The detector may be provided in the BSA 12, may also be provided in the collimator 13, and may further be provided at any position close to the BSA 12, provided that the detector can detect the neutron dose of the neutron beam.

For the detector capable of detecting the neutron dose of the neutron beam in real time, there are an ionization chamber and a scintillator detector. The He$^{-3}$ proportional counter, the BF$_3$ proportional counter, the fission chamber, and the boron ionization chamber take a structure of the ionization chamber as a substrate. The scintillator detector includes an organic material or an inorganic material. In detection on the thermal neutrons, a high thermal neutron capture cross-section element such as Li or B is added to the scintillator detector in most cases. A capture or fission reaction occurs between some element in the detector and the neutron entering the detector to release heavy charged particles and fission fragments, thereby forming a large number of ion pairs in the ionization chamber or the scintillator detector. Charges are acquired to form an electrical signal. Through noise reduction, conversion, and separation of the signal processing unit, the electrical signal is converted into a pulse signal. By analyzing a voltage pulse, a neutron pulse signal and a gamma pulse signal are discriminated. The neutron pulse signal is recorded continuously by the counter to form a neutron count rate (n/s). The conversion unit is configured to perform operation and conversion on the count rate through internal software, an internal program and the like to obtain the neutron flux rate (cm$^{-2}$s$^{-1}$). Through further operation and conversion on the neutron flux rate, the neutron dose rate (Gy/s) is obtained. At last, an integrating portion is configured to perform integration on the neutron dose rate to obtain a real-time neutron dose.

Hereinafter, the fission chamber, the scintillator detector, and the BF$_3$ proportional counter are used as an example for simple description.

When passing through the fission chamber, the neutron beam is ionized with a gas molecule in the fission chamber or a wall of the fission chamber to generate an electron and a positively charged ion. The electron and the positively charged ion are referred to as the above ion pair. Due to a high-voltage electric field applied to the fission chamber, the electron moves toward a central anode wire, and the positively charged ion moves toward a cathode wall, thereby generating a measurable electrical signal.

Substances such as optical fibers in the scintillator detector absorb energies to generate visible light, which excites the electron in the crystal or the molecule by means of ionizing radiation. Fluorescence generated by the electron in a ground state is acquired to detect the neutron beam. The visible light generated by interaction between the scintillator detector and the neutron beam is converted into an electrical signal through a photomultiplier.

The BF$_3$ proportional counter is provided in the BSA and configured to receive irradiation of the neutron beam. A $^{10}$B(n, alpha)$^7$Li nuclear reaction occurs between the element B in the BF$_3$ proportional counter and the neutron. Under driving of a voltage, the alpha particle and the $^7$Li charged particle generated by the nuclear reaction are acquired by a high-voltage electrode to generate the electrical signal. The electrical signal is transmitted to the signal processing unit through a coaxial cable. Through amplification, filtration and shaping, a pulse signal is formed. The processed pulse signal is transmitted to the counter for pulse counting, thereby obtaining a count rate (n/s). An intensity of the neutron beam, namely a neutron dose, can be measured in real time through the count rate.

In an embodiment of the present disclosure, the BF$_3$ proportional counter is preferably used to detect the neutron dose. Certainly, there are no limits made on the type of the detector, provided that the neutron dose can be detected in real time.

Before the neutron beam is irradiated onto the irradiated body S, the blood boron concentration detecting device 4 is required to detect the actual blood boron concentration of the irradiated body. According to the actual blood boron concentration, the preset irradiation dose is corrected. The boron concentration may be detected by an inductively coupled plasma atomic emission spectroscopy (ICP-AES), a high-resolution alpha-particle autoradiography, a charged ion spectroscopy, a neutron capture camera, an MRI, a PET, an instantaneous gamma-ray spectrometer, etc. The device involved in the above detection methods is called the boron concentration detecting device.

The placement module 7 includes a placement table configured to support the irradiated body S and a drive portion configured to drive the placement table to move to an irradiating position.

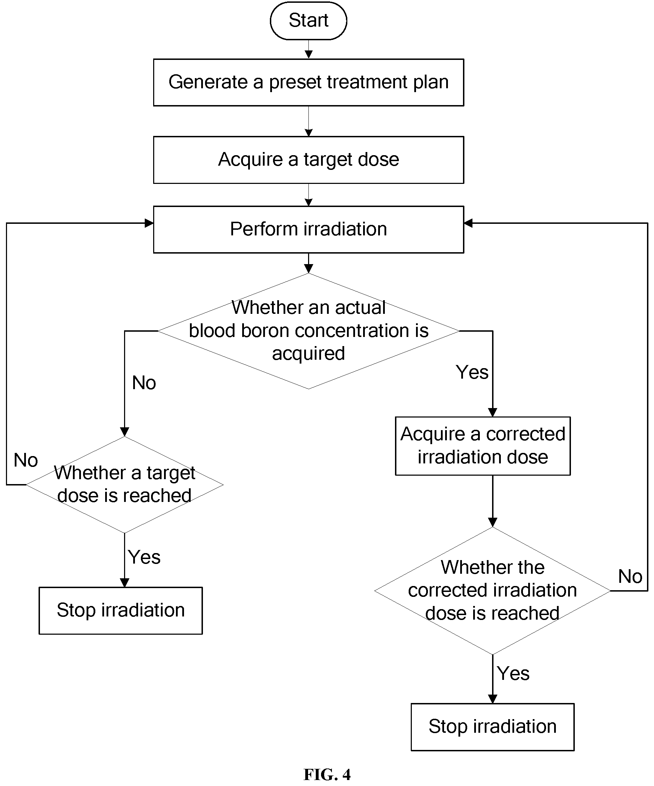

Referring to FIG. 4, an irradiation dose correction method for a BNCT system 100 in the embodiment of the present disclosure includes the following steps:

S1: A preset treatment plan is generated.

The preset treatment plan is generated in combination with a preset boron concentration and medical image data of an irradiated body. The preset treatment plan includes preset irradiation parameters such as preset irradiation time $T_{preset}$, and a preset target dose rate.

A target dose $D_{ROI,prescribed}$, or a prescribed dose, is set by a medical staff in combination with a body characteristic parameter of the irradiated body, the medical image data of the irradiated body and working experience. The target dose $D_{ROI,prescribed}$ is a neutron dose actually to be received by the irradiated body.

In other embodiments, the above parameters or more unmentioned parameters may be understood as the preset irradiation parameters.

S2: A preset irradiation dose in the preset treatment plan is acquired.

In the embodiment, a cumulative neutron count is taken as a monitoring parameter of an online monitoring system for an irradiation dose in the BNCT. Through calculation and correction on the cumulative neutron count, the irradiation dose is corrected. When the cumulative neutron count reaches a target value, the irradiation dose is the target dose.

A preset cumulative neutron count N$_{preset}$ is calculated by:

$$N_{preset} = R_{BF3,cal} \times T_{preset} \qquad (1)$$

In the foregoing equation, R$_{BF3,cal}$ is a theoretical count rate of a neutron dose detecting device 3, and T$_{preset}$ is the preset irradiation time corresponding to the preset boron concentration B$_{preset}$, and is calculated by:

$$T_{preset} = \frac{D_{ROI,prescribed}}{\dot{D}_{ROI}(B_{preset} \times TBR)} \qquad (2)$$

The preset target dose rate $\dot{D}_{ROI}(B_{preset} \times TBR)$ is calculated by:

$$\dot{D}_{ROI}(B_{preset} \times TBR) = aB_{preset}^2 + bB_{preset} + c \qquad (3)$$

In the foregoing equation, a, b, and c each are a fitting coefficient, which is obtained through function fitting on a plurality of preset boron concentrations and corresponding simulated dose rates.

S3: Irradiation is performed on an irradiated body S according to the preset treatment plan, and an actual boron concentration and a corrected irradiation dose are acquired in the irradiation.

In an embodiment of the present disclosure, the irradiation dose is corrected according to the actually detected boron concentration to obtain the corrected irradiation dose. In the embodiment, a corrected cumulative neutron count N$_{update}$ is acquired to determine whether the target dose is reached. The blood boron concentration of the irradiated body is detected by a blood boron concentration detecting device 4. After the actual blood boron concentration B$_{update}$ is acquired, the corrected cumulative neutron count N$_{update}$ corresponding to the actual blood boron concentration B$_{update}$ is calculated by:

$$N_{update} = R_{BF3,cal} \times T_{update} = R_{BF3,cal} \times \frac{D_{ROI,prescribed}}{\dot{D}_{ROI}(B_{update} \times TBR)} \qquad (4)$$

In the foregoing equation, T$_{update}$ is a planned irradiation time corresponding to the actual blood boron concentration B$_{update}$, $\dot{D}_{ROI}(B_{update} \times TBR)$ is the corrected target dose rate corresponding to the actual blood boron concentration B$_{update}$, and $\dot{D}_{ROI}(B_{update} \times TBR)$ is calculated by:

$$\dot{D}_{ROI}(B_{update} \times TBR) = aB_{update}^2 + bB_{update} + c \qquad (5)$$

In the foregoing equation, a, b, and c each are a fitting coefficient, which is obtained through function fitting on a plurality of preset boron concentrations and corresponding simulated dose rates.

S4: The irradiation is controlled according to the corrected irradiation dose.

Whether the irradiation dose received by the irradiated body S reaches the target dose is determined according to the corrected irradiation dose. When the cumulative neutron count detected by the neutron dose detecting device 3 reaches the corrected cumulative neutron count N$_{update}$, the irradiation dose received by the irradiated body S reaches the target dose D$_{ROI,prescribed}$, and a control module 6 controls a neutron beam irradiation module 1 to stop the irradiation.

It may be understood that if the actual boron concentration is not acquired all the time in treatment, but the neutron dose detected by the neutron dose detecting device 3 reaches the preset target dose, the irradiation dose received by the irradiated body reaches the prescribed dose D$_{ROI,prescribed}$, and the control module 6 controls the neutron beam irradiation module 1 to stop the irradiation.

In an embodiment of the present disclosure, in actual irradiation, the neutron dose detecting device 3 detects the irradiation dose of the neutron beam in real time. When the neutron irradiation dose reaches the corrected irradiation dose or the preset target dose, the irradiation is stopped. In other implementations, the irradiation may be controlled by monitoring irradiation time. Specifically, target irradiation time is calculated based on the corrected irradiation dose or the preset target dose. When actual irradiation time reaches target irradiation time, the control module 6 controls the neutron beam irradiation module 1 to stop the irradiation. That is, the irradiation may be controlled based on the target irradiation time in the step S4.

The target irradiation time T is calculated by:

$$T = \frac{R_{BF3,cal}}{R_{BF3,QC}} \times T_1 \qquad (6)$$

In the foregoing equation, $R_{BF3,cal}$ is a count rate of a $BF_3$ proportional counter under a given reference radioactive source, $R_{BF3,QC}$ is a $BF_3$ count rate measured in daily beam quality control (QC), and reflects a $BF_3$ neutron count rate at an irradiation day, and $T_1$ is irradiation time corresponding to a blood boron concentration $B_1$, and is calculated by:

$$T_1 = \frac{D_{ROI,prescribed}}{\dot{D}_{ROI}(B_1 \times TBR)} \qquad (7)$$

$$\dot{D}_{ROI}(B_1 \times TBR) = aB_1^2 + bB_1 + c \qquad (8)$$

In an embodiment of the present disclosure, before the irradiation is performed, the blood boron concentration detecting device 4 is used to detect the actual blood boron concentration of the irradiated body. According to the actual blood boron concentration before the irradiation is performed on the irradiated body, corrected irradiation time is calculated. In other implementations, since actual irradiation parameters change in irradiation, target irradiation parameters are to be adjusted periodically or in time according to a specific condition, so as to ensure the treatment effect to the utmost extent. When the neutron beam is irradiated onto the irradiated body S, a boron-containing drug is supplied continuously to the irradiated body S. However, a boron concentration in the irradiated body is hardly maintained at a same level all the time in the whole irradiation. Referring to Eq. 5, the target dose rate changes with the blood boron concentration. While the prescribed dose, namely the target dose, is unchanged, when the blood boron concentration changes, both the irradiation dose and the target irradiation time are further corrected to ensure that the neutron dose actually received by the irradiated body is the same as the prescribed dose to guarantee the treatment effect.

Accordingly, a step S5 may further be included in an embodiment of the present disclosure. The blood boron concentration of the irradiated body is detected in real time or periodically in the actual irradiation. The irradiation dose or the target irradiation time is corrected in real time or periodically according to the detected blood boron concentration. The steps S3-S4 are repeated, until the neutron dose detected by the neutron dose detecting device 3 reaches the target dose or the actual irradiation time is the same as the corrected target irradiation time. On the other hand, parameters of the neutron beam generated by the neutron irradiation module may also change. The neutron dose detecting device 3 can monitor the parameters of the neutron beam in real time. When the parameters of the neutron beam change, the target irradiation time may also be corrected appropriately to ensure that the neutron dose actually received by the irradiated body is the same as the prescribed dose.

There are many methods for detecting the blood boron concentration of the irradiated body in real time. In an embodiment of the present disclosure, a case where the boron concentration of the irradiated body S is calculated by detecting gamma rays released by the irradiated body S is used as an example for description. The neutron beam in the irradiated body is reacted with the boron to generate the gamma rays. By measuring an amount of the gamma rays, an amount of the boron reacting with the neutron beam can be calculated, thereby obtaining the boron concentration in the irradiated body S. Specifically, the boron concentration detecting device detects the gamma rays (478 kev) generated by the neutron and the boron in reaction, thereby measuring the boron concentration. A boron distribution measuring system (PG(Prompt-y)-SPECT) capable of measuring monokinetic gamma rays to obtain a boron concentration distribution is used as the boron concentration detecting device. The boron concentration detecting device is provided with a gamma-ray detecting portion and a boron concentration calculating portion. The gamma-ray detecting portion is configured to detect information associated with the gamma rays released from the irradiated body S. The boron concentration calculating portion is configured to calculate the boron concentration of the irradiated body S according to the information detected by the gamma-ray detecting portion and associated with the gamma rays. The gamma-ray detecting portion can use a scintillator and other various gamma-ray detecting devices. In the implementation, the gamma-ray detecting portion is provided near a tumor of the irradiated body S, for example, at a position with about 30 cm away from the tumor of the irradiated body S.

According to the BNCT system 100 provided by the present disclosure, by performing simulation according to the preset blood boron concentration to obtain the preset treatment plan, obtaining the preset target dose, performing calculation according to the dose rate corresponding to the actual blood boron concentration to obtain the corrected irradiated dose, and controlling the neutron beam irradiation module to implement the irradiation, there is no need to inject the boron-containing drug into the irradiated body when the preset treatment plan is formulated, as well as no need to implement the irradiation after the blood boron concentration is detected. This saves the treatment cost, and simplifies the treatment process. By correcting the target irradiation dose according to the actual blood boron concentration in treatment, an error in the irradiation dose due to a change of the blood boron concentration in the actual irradiation is reduced.

It should be understood that although the steps in the flowcharts in the above embodiments are shown in sequence as indicated by the arrows, these steps are not necessarily performed in sequence as indicated by the arrows. The execution order of these steps is not strictly limited, and these steps may be executed in other orders, unless clearly described otherwise. Moreover, at least some of the steps in the flowcharts in the above embodiments may include a plurality of steps or stages. The steps or stages are unnecessarily executed at the same time, but may be executed at different times. The execution order of the steps or stages is unnecessarily carried out sequentially, but may be executed alternately with other steps or at least some of the steps or stages of other steps.

The technical characteristics of the above embodiments can be employed in arbitrary combinations. To provide a concise description of these embodiments, all possible combinations of all the technical characteristics of the above embodiments may not be described; however, these combinations of the technical characteristics should be construed as falling within the scope defined by the specification as long as no contradiction occurs.

The above embodiments are merely illustrative of several implementations of the present disclosure, and the description thereof is more specific and detailed, but is not to be construed as a limitation to the patentable scope of the present disclosure. It should be noted that those of ordinary skill in the art can further make variations and improvements without departing from the conception of the present disclosure. These variations and improvements all fall within the protection scope of the present disclosure. Therefore, the protection scope of the present disclosure should be subject to the protection scope defined by the appended claims.

**Claims**

1. A boron neutron capture therapy (BNCT) system, comprising:

    a neutron beam irradiation module configured to generate a neutron beam;
    a blood boron concentration detecting device configured to detect an actual blood boron concentration of an irradiated body;
    a treatment planning module configured to generate a preset treatment plan;
    an irradiation dose correction module configured to acquire a corrected irradiation dose according to the actual blood boron concentration; and
    a control module configured to invoke the preset treatment plan from the treatment planning module, and control irradiation time of the neutron beam irradiation module according to the corrected irradiation dose, such that an irradiation dose received by a patient reaches a target dose.

2. The BNCT system according to claim 1, wherein the treatment planning module is configured to perform simulation and calculation according to a preset blood boron concentration to obtain the preset treatment plan.

3. The BNCT system according to claim 2, wherein the irradiation dose correction module is configured to correct a target dose rate according to the actual blood boron concentration to obtain the corrected irradiation dose.

4. The BNCT system according to claim 3, further comprising a neutron dose detecting device configured to detect a cumulative neutron count in real time to obtain the irradiation dose.

5. The BNCT system according to claim 4, wherein the neutron dose detecting device is a $BF_3$ proportional counter.

6. The BNCT system according to claim 5, wherein a preset cumulative neutron count $N_{preset}$ is calculated by:

$$N_{preset} = R_{BF3,cal} \times T_{preset} \tag{1}$$

wherein, $R_{BF3,cal}$ is a theoretical count rate of the neutron dose detecting device, $B_{preset}$ is the preset boron concentration, $T_{preset}$ is preset irradiation time corresponding to the preset boron concentration $B_{preset}$, and $T_{preset}$ is calculated by:

$$T_{preset} = \frac{D_{ROI,prescribed}}{\dot{D}_{ROI}(B_{preset} \times TBR)} \qquad (2)$$

wherein, $D_{ROI,prescribed}$ is the target dose, $\dot{D}_{ROI}(B_{preset} \times TBR)$ is a preset target dose rate, and $\dot{D}_{ROI}(B_{preset} \times TBR)$ is calculated by:

$$\dot{D}_{ROI}(B_{preset} \times TBR) = aB^2_{preset} + bB_{preset} + c \qquad (3)$$

wherein, a, b, and c each are a fitting coefficient, which is obtained through function fitting on a plurality of preset boron concentrations and corresponding simulated dose rates.

7. The BNCT system according to claim 4, wherein a corrected cumulative neutron count $N_{update}$ is calculated by:

$$N_{update} = R_{BF3,cal} \times T_{update} = R_{BF3,cal} \times \frac{D_{ROI,prescribed}}{\dot{D}_{ROI}(B_{update} \times TBR)} \qquad (4)$$

wherein, $B_{update}$ is the actual blood boron concentration, $T_{update}$ is a planned irradiation time corresponding to the actual blood boron concentration $B_{update}$, $\dot{D}_{ROI}(B_{update} \times TBR)$ is a corrected target dose rate corresponding to the actual blood boron concentration $B_{update}$, and the $\dot{D}_{ROI}(B_{update} \times TBR)$ is calculated by:

$$\dot{D}_{ROI}(B_{update} \times TBR) = aB^2_{update} + bB_{update} + c \qquad (5)$$

wherein, a, b, and c each are a fitting coefficient, which is obtained through function fitting on a plurality of preset boron concentrations and corresponding simulated dose rates.

8. An irradiation dose correction method for a boron neutron capture therapy (BNCT) system, comprising:

generating a preset treatment plan in combination with a preset boron concentration and medical image data of an irradiated body;
acquiring a corrected irradiation dose according to an actual boron concentration; and
controlling irradiation time according to the corrected irradiation dose, such that an irradiation dose received by a patient reaches a target dose.

9. The irradiation dose correction method according to claim 8, further comprising: determining the target dose based on the medical image data of the irradiated body.

10. The irradiation dose correction method according to claim 9, further comprising: acquiring a preset irradiation dose.

11. The irradiation dose correction method according to claim 10, wherein acquiring the corrected irradiation dose specifically comprises: correcting a preset target dose rate in the preset treatment plan according to the actually detected boron concentration to obtain a corrected target dose rate; and performing calculation according to the corrected target dose rate to obtain the corrected irradiation dose.

12. The irradiation dose correction method according to claim 10, wherein a preset cumulative neutron count $N_{preset}$ is calculated by:

$$N_{preset} = R_{BF3,cal} \times T_{preset} \qquad (1)$$

wherein, $R_{BF3,cal}$ is a theoretical count rate of a neutron dose detecting device, $B_{preset}$ is the preset boron concentration, $T_{preset}$ is preset irradiation time corresponding to the preset boron concentration $B_{preset}$, and $T_{preset}$ is calculated by:

$$T_{preset} = \frac{D_{ROI,prescribed}}{\dot{D}_{ROI}(B_{preset} \times TBR)} \qquad (2)$$

wherein, $D_{ROI,prescribed}$ is the target dose, $\dot{D}_{ROI}(B_{preset} \times TBR)$ is a preset target dose rate, and $\dot{D}_{ROI}(B_{preset} \times TBR)$ is calculated by:

$$\dot{D}_{ROI}(B_{preset} \times TBR) = aB_{preset}^2 + bB_{preset} + c \qquad (3)$$

wherein, a, b, and c each are a fitting coefficient, which is obtained through function fitting on a plurality of preset boron concentrations and corresponding simulated dose rates.

13. The irradiation dose correction method according to claim 11, wherein a corrected cumulative neutron count $N_{update}$ is calculated by:

$$N_{update} = R_{BF3,cal} \times T_{update} = R_{BF3,cal} \times \frac{D_{ROI,prescribed}}{\dot{D}_{ROI}(B_{update} \times TBR)} \qquad (4)$$

wherein, $B_{update}$ is the actual blood boron concentration, $T_{update}$ is a planned irradiation time corresponding to the actual blood boron concentration $B_{update}$, $\dot{D}_{ROI}(B_{update} \times TBR)$ is the corrected target dose rate corresponding to the actual blood boron concentration $B_{update}$, and the $\dot{D}_{ROI}(B_{update} \times TBR)$ is calculated by:

$$\dot{D}_{ROI}(B_{update} \times TBR) = aB_{update}^2 + bB_{update} + c \qquad (5)$$

wherein, a, b, and c each are a fitting coefficient, which is obtained through function fitting on a plurality of preset boron concentrations and corresponding simulated dose rates.

14. The irradiation dose correction method according to claim 11, further comprising: providing a neutron dose detecting device configured to detect the irradiation dose in real time; and when the irradiation dose detected by the neutron dose detecting device reaches the corrected irradiation dose, controlling, by a control module, a neutron beam irradiation module to stop irradiation.

15. The irradiation dose correction method according to claim 14, wherein the neutron dose detecting device is a $BF_3$ proportional counter.

100

Neutron beam irradiation module — 1

Image acquisition module — 2

Neutron dose detecting device — 3

Blood boron concentration detecting device — 4

Treatment planning module — 5

Control module — 6

Placement module — 7

Irradiation dose correction module — 8

FIG. 1

**FIG. 2**

**FIG. 3**

**FIG. 4**

## INTERNATIONAL SEARCH REPORT

| | International application No. |
|---|---|
| | **PCT/CN2023/129479** |

| A. | CLASSIFICATION OF SUBJECT MATTER |
|---|---|

A61B 5/145(2006.01)i; A61N 5/10(2006.01)i; G01T 1/02(2006.01)i; G16H20/40(2018.01)i

According to International Patent Classification (IPC) or to both national classification and IPC

| B. | FIELDS SEARCHED |
|---|---|

Minimum documentation searched (classification system followed by classification symbols)

IPC: A61B A61N G01T G16H

Documentation searched other than minimum documentation to the extent that such documents are included in the fields searched

Electronic data base consulted during the international search (name of data base and, where practicable, search terms used)

CNABS; CNTXT; CJFD; ENTXTC; ENTXT; VEN; CNKI; IEEE: 中硼, 中子, 剂量, 计数, 血硼, 硼浓度, 治疗计划, 修正, 校正, boron, neutron, dosage, count+, concentration, correct+, modified, modification

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 113877079 A (NEUBORON THERAPY SYSTEM LTD.) 04 January 2022 (2022-01-04) description, paragraphs [0004]-[0168], and figures 1-7 | 1-7 |
| X | CN 113877077 A (NEUBORON THERAPY SYSTEM LTD.) 04 January 2022 (2022-01-04) description, paragraphs [0004]-[0175], and figures 1-7 | 1-7 |
| X | CN 113877081 A (NEUBORON THERAPY SYSTEM LTD.) 04 January 2022 (2022-01-04) description, paragraphs [0004]-[0166], and figures 1-7 | 1-7 |
| X | CN 113877076 A (NEUBORON THERAPY SYSTEM LTD.) 04 January 2022 (2022-01-04) description, paragraphs [0004]-[0179], and figures 1-7 | 1-7 |
| X | CN 113877075 A (NEUBORON THERAPY SYSTEM LTD.) 04 January 2022 (2022-01-04) description, paragraphs [0004]-[0165], and figures 1-7 | 1-7 |
| A | TW 202112414 A (SUMITOMO HEAVY INDUSTRIES, LTD.) 01 April 2021 (2021-04-01) entire document | 1-7 |
| A | US 2018326225 A1 (NEUBORON MEDTECH LTD.) 15 November 2018 (2018-11-15) entire document | 1-7 |

☐ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **25 January 2024** | **04 February 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/129479**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **8-15**
because they relate to subject matter not required to be searched by this Authority, namely:

Claims 8-15 set forth an irradiation dose correction method for a boron neutron capture therapy system. Claims 8-15 comprise the step of "controlling irradiation time according to the corrected irradiation dose, so that an irradiation dose received by a patient reaches a target dose", and the step belongs to a treatment step; and claims 14 and 15 further comprise the step "when an irradiation dose detected by a neutron dose detection device reaches the corrected irradiation dose, a control module controlling a neutron beam irradiation module to stop irradiation", and the step also belongs to a treatment step. It can be seen therefrom that claims 8-15 relate to a treatment method, and therefore fall within subject matter for which no search is required by the International Searching Authority as defined in PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

| Patent document cited in search report | | | Publication date (day/month/year) | Patent family member(s) | | | Publication date (day/month/year) |
|---|---|---|---|---|---|---|---|
| CN | 113877079 | A | 04 January 2022 | None | | | |
| CN | 113877077 | A | 04 January 2022 | None | | | |
| CN | 113877081 | A | 04 January 2022 | None | | | |
| CN | 113877076 | A | 04 January 2022 | None | | | |
| CN | 113877075 | A | 04 January 2022 | None | | | |
| TW | 202112414 | A | 01 April 2021 | None | | | |
| US | 2018326225 | A1 | 15 November 2018 | US | 10537750 | B2 | 21 January 2020 |

Form PCT/ISA/210 (patent family annex) (July 2022)